# EUROPEAN PATENT APPLICATION

(11) **EP 2 033 632 A1**
(43) Date of publication of application: **11.03.2009**
(21) Application number: 07115985.9
(22) Date of filing: 10.09.2007
(51) Int. Cl.: A61K 31/00, A61K 31/46, A61P 9/10

(54) **5-HT3 receptor antagonists for the teatment of myocardial infarction, stroke, thrombosis and atherosclerosis**

(71) Applicant: Novasearch AG, 6304 Zug (CH)
(72) Inventor: Trenk, Dietmar, 79219, Staufen (DE); Stratz, Christian, 79111, Freiburg (DE); Stratz, Thomas, 79713, Bad Säckingen (DE); Fiebich, Bernd, 79111, Freiburg (DE); Müller, Wolfgang, 4102, Binningen (CH)
(74) Representative: Becker, Konrad

(57) **Abstract**

5-HT₃ receptor antagonists are useful in the treatment of a disease caused or influenced by activation of thrombocytes, in particular myocardial infarction, stroke, thrombosis and atherosclerosis.

## Description

This invention relates to a new use of 5-HT₃ receptor antagonists for the treatment of diseases caused or influenced by activation of thrombocytes.

5-HT₃ receptor antagonists are a class of compounds which block 5-HT₃ receptors, and are also sometimes classified as serotonin M receptor antagonists. The 5-HT₃ receptor antagonists comprise a defined and recognised class of pharmaceutically active compounds well known in the art and characterised, as their name implies, by their pharmacological activity. Various 5-HT₃ receptor antagonist compounds are commercially available and clinically applied, e.g. in the treatment of emesis.

5-HT₃ receptor antagonists from various sources have been published for a wide variety of uses, for example for the treatment of visceral pain, migraine, vascular and cluster headache, trigeminal neuralgia, arrhythmia, serotonin-induced gastro-intestinal disorders, including emesis induced by anti-cancer agents, anxiety, stress-related psychiatric disorders, depression, cognitive disorders, social withdrawal, panic attacks, agoraphobia, lung embolism, rhinitis or serotonin-induced nasal disorders, fibromyalgia and local treatment of pain caused by various non-inflammatory or inflammatory conditions. Some have been commercially introduced for the treatment of emesis.

In accordance with the present invention it has now surprisingly been found that 5-HT₃ receptor antagonists are useful for the treatment of diseases caused or influenced by activation of thrombocytes.

Thrombocytes play a central role in blood coagulation (clotting) and are therefore also of high importance in the pathogenesis of cardiac infarction and stroke, furthermore also in thrombosis of the veins and inflammatory conditions in the development of atherosclerosis.

Activation of thrombocytes causing blood clotting is based on several mechanisms. It has now been demonstrated that 5-HT₃ receptors are present on platelets and that the number of these receptors at the platelet surface is increasing in a dose dependent fashion on addition of ADP (adenosine diphosphate) or TRAP (thrombin receptor activating peptide) known to stimulate thrombocyte activation. The increase of 5-HT₃ receptors on addition of compounds inducing aggregation such as ADP and TRAP is proof that such receptors play a role in thrombotic processes.

Platelet activity is also important in inflammatory processes in atherosclerotic conditions. Thrombocytes activated by thrombin may, as is already known, induce the production of IL-1β, IL-8, MCP (monocyte chemoattractant protein) and other inflammation mediators These are impeded by 5-HT₃ receptor antagonists. This demonstrates that 5-HT₃ receptor antagonists not only influence blood coagulation but also processes playing a role in the development of atherosclerosis.

Hence, the present invention relates to the use of a 5-HT₃ receptor antagonist or of a pharmaceutically acceptable salt of such an antagonist for the manufacture of a pharmaceutical composition for the treatment of a disease caused or influenced by activation of thrombocytes, in particular myocardial infarction, stroke, thrombosis and atherosclerosis.

Any 5-HT₃ receptor antagonist can be used in accordance with the invention. Preferred 5-HT₃ receptor antagonists which may be employed in accordance with the present invention are ondansetron, 1,2,3,9-tetrahydro-9-methyl-3-[(2-methyl-1H-imidazol-1-yl)-methyl]-4H-carbazol-4-one (cf. Merck Index, twelfth edition, item 6979), granisetron, endo-1-methyl-N-(9-methyl-9-aza-bicyclo[3.3.1]non-3-yl)-1H-imidazole-3-carboxamide (cf. loc. cit., item 4557), or dolasetron, 1H-indole-3-carboxylic acid (2α,6α,8α,9αβ)-octahydro-3-oxo-2,6-methano-2H-quinolizin-8-yl ester, (cf. loc. cit., item 3471).

Particular 5-HT₃ receptor antagonists which may be employed in accordance with the present invention are those of the formula 1 as defined in European Patent Publication EP 0 189 002 B1, in particular tropisetron, indol-3-yl-carboxylic acid-endo-8-methyl-8-aza-bicyclo[3,2,1]-oct-3-yl-ester, (cf. loc. cit., item 9914), ramosetron, 4,5,6,7-tetrahydro-5-[(1-methyl-indol-3-yl)carbonyl]benzimidazole (U.S. Pat. No. 5,344,927), fabesetron, (+)-10-methyl-7-(5-methyl-1H-imidazol-4-ylmethyl)-6,7,8,9-tetrahydropyrido[1,2-a]indol-6-one (EP 0 361 317), lintopride, N-(1-ethyl-2-imidazolin-2-y-methyl)-2-methoxy-4-amino-5-chlorobenzamide (Chem. Abstr. No. 107429-63-0), alosetron, 2,3,4,5-tetrahydro-5-methyl-2-[(5-methyl-1H-imidazol-4-yl)methyl]-1H-pyrido[4,3-b]indol-1-one (EP 0 306 323), cilansetron, (-)-(R)-5,6,9,10-tetrahydro-10-[(2-methylimidazol-1-yl)methyl]-4H-pyrido-(3,2,1-jk)carbazol-11(8H)-one, palonosetron, 2-(3S)-1-azabicyclo[2.2.2]oct-3-yl-2,3,3a(S), 4,5,6-hexahydro-1H-benz(de)isoquinolin-1-one, azasetron, N-(1-azabicyclo[2.2.2]octan-8-yl)-6-chloro-4-methyl-3-oxo-1,4-benzoxazine-8-carboxamide, and zatosetron, 5-chloro-2,2-dimethyl-N-(8-methyl-8-azabicyclo[3.2.1]octan-3-yl)-3H-1-benzofuran-7-carboxamide.

5-HT₃ receptor antagonists may be employed in accordance with the invention in free or in pharmaceutically acceptable salt form, e.g. as known in the art, for example, in the case of compounds mentioned above in pharmaceutically acceptable acid addition salt form, for example, in the case of ondansetron as the hydrochloride dihydrate, granisetron the hydrochloride, dolasetron the mesylate, tropisetron the monohydrochloride, ramosetron fabesetron, alosetron and cilansetron the hydrochlorides, palonosetron the monohydrochloride, azasetron the hydrochloride, and zatosetron the maleate.

References to 5-HT₃ receptor antagonists collectively or individually throughout the present specification and claims are accordingly to be understood as embracing both free compounds and such pharmaceutically acceptable salt forms, e.g. as clinically employed, and further also solvates, e.g. hydrates, or specific crystal forms of any of these compounds or salts.

For use in accordance with the present invention tropisetron (especially in the formulation called Navoban^{™}) is most preferred.

Thus, the invention relates to the use of a 5-HT₃ receptor antagonist or of a pharmaceutically acceptable salt of such an antagonist for the manufacture of a pharmaceutical composition for the treatment of a disease caused or influenced by activation of thrombocytes, in particular myocardial infarction, stroke, thrombosis and atherosclerosis, where the 5-HT₃ receptor antagonist is selected from the group consisting of ondansetron, granisetron, dolasetron, tropisetron, ramosetron, fabesetron, lintopride alosetron, cilansetron, palonosetron, azasetron and zatosetron, which may be used in free form or as a pharmaceutically acceptable salt.

"Treatment" as used herein includes use for the alleviation, amelioration or control of said diseases, processes, conditions or events. It also includes intervention for the alleviation, amelioration or control of the sequelae or symptoms of any one or more of these diseases, for example myocardial infarction, stroke, thrombosis and atherosclerosis. In this context the term "treatment" is further to be understood as embracing use to reverse, restrict or control progression of any specified disease, process, condition event or the like, including use for disease modifying effect. The present invention is in particular applicable to the treatment of myocardial infarction and stroke.

Treatment is preferably by enteral, especially peroral application, e.g. by use of tablets or capsules, or rectal application, e.g. by use of enemation or suppositories, or by subcutaneous, intraperitoneal or intra-muscular injection or infusion.

From the foregoing it will be noticed that the present invention is to be understood especially as embracing the treatment, e.g. therapy, of any disease, process, symptom, event or condition caused or influenced by activation of thrombocytes.

For this indication the appropriate dosage will, of course, vary depending upon, for example, the compound employed, the host, the mode of administration and the nature and severity of the condition being treated. An indicated daily dosage is in the range usually used for known indications such as emesis and is typically from about 0.05 to about 50 mg, conveniently administered, for example, in divided doses up to four times a day, in unit dosage form or in sustained release form.

The compounds of the invention may be administered by any conventional route, in particular enterally, preferably orally e.g. in the form of tablets or capsules, or parenterally, e.g. in the form of injectable solutions or suspensions.

The present invention also provides pharmaceutical compositions comprising the compounds in association with at least one pharmaceutical carrier or diluent for use in the treatment of diseases caused or influenced by activation of thrombocytes, in particular myocardial infarction, stroke, thrombosis and atherosclerosis. Such compositions may be manufactured in conventional manner. Unit dosage forms may contain for example from about 0.01 mg to about 25 mg of the compound.

The invention furthermore provides a method for the treatment of diseases caused or influenced by activation of thrombocytes, in particular myocardial infarction, stroke, thrombosis and atherosclerosis, in a subject in need of such treatment, which comprises administering to said subject a therapeutically effective amount of a compound of the invention.

In further aspects the present invention also provides a 5-HT₃ receptor antagonist for use in the manufacture of a pharmaceutical composition for the treatment of diseases caused or influenced by activation of thrombocytes, in particular myocardial infarction, stroke, thrombosis and atherosclerosis.

The 5-HT₃ receptor antagonists are preferably used in well-known liquid formulations.

In a further aspect it has been found in accordance with the present invention that 5-HT₃ receptor antagonists are useful as part of a combination therapy in the treatment of diseases caused or influenced by activation of thrombocytes, in particular myocardial infarction, stroke, thrombosis and atherosclerosis.

Where co-administration is practiced the drug substances, i.e. the 5-HT₃ receptor antagonist and the standard drug to treat myocardial infarction, stroke, thrombosis and atherosclerosis, may be administered sequentially or simultaneously or substantially simultaneously, e.g. employing a fixed combination dosage form.

In further aspects the present invention also provides a pharmaceutical dosage form comprising a 5-HT₃ receptor antagonist together with a standard drug to treat myocardial infarction, stroke, thrombosis and atherosclerosis.

Dosage forms are to be understood as including both fixed-unit-dosage forms, e.g. liquid formulations, comprising both active ingredients together with appropriate pharmaceutically acceptable diluents or carriers, as well as twin delivery systems, packages or the like comprising both active ingredients separately or in separate dosage form, for concomitant or sequential administration.

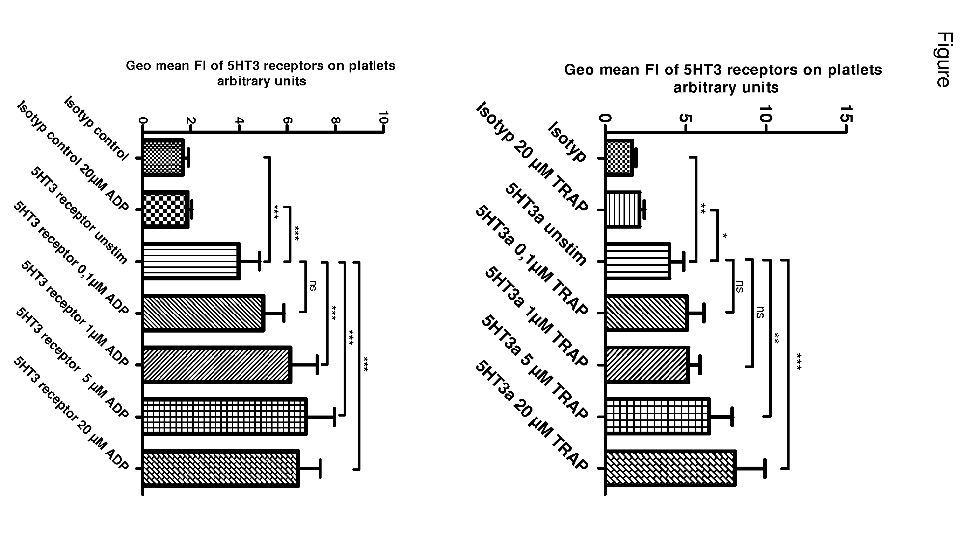

### Brief description of the Figure:

Geometric mean florescence (measured with a BD FACSscan flow cytometer) of 5-HT₃ receptor expression on platelets surface (primary 5-HT₃ₐ antibodies coupled with FITC labeled IgG antibodies); values are mean ± standard deviation from 7 different young healthy volunteers (5 male, 2 female); Gauss distribution was tested by the Kolmogorow-Smirnov test and one way ANOVA test including Bonferroni's Multiple Comparison test; * p < 0,05 ** p < 0,01 *** p < 0,001.
(upper panel) 5-HT₃ receptor expression native and after platelet stimulation with ADP in different concentrations for 15 min; isotype control was done with the secondary antibody (IgG FITC labeled; specific for the 5-HT₃ₐ receptor)
(lower panel) 5-HT₃ receptor expression native and after platelet stimulation with TRAP in different concentrations for 15 min; isotype control was done as by the ADP stimulation.

It was examined, whether platelets contain 5-HT₃ receptors and if the 5-HT₃ receptor surface expression is modulated by the activation of platelets with ADP and TRAP: Platelets were isolated and incubated with increasing doses of ADP (0.1 µM - 20 µM) and TRAP (0.1 - 20 µM) for 15 min. FACS analysis was performed using an anti 5-HT₃ₐ antibody (Genway) to characterize the surface expression of 5-HT₃ₐ on platelets (in combination with a secondary FITC labeled IgG antibody specific against the 5-HT₃ₐ receptor antibody). Furthermore, Western blot technique was used to confirm the presence of 5-HT₃ₐ in platelets.

5-HT₃ immunoreactivity on unstimulated platelets is found, which is dose-dependently increased by activation with ADP (Figure, upper panel). 1 µM and higher doses of ADP showed a highly significant increase in 5-HT₃ on platelets. A comparable effect was observed by using TRAP as platelet activator (Figure, lower panel) resulting in significant increasing effects with the doses of 5 and 20 µM. The presence of 5-HT_{3A} receptors on platelets was further confirmed by Western blots, showing, strong immunoreactivity at the respective molecular weight of 55 kDa in platelets of three different donors.

## Claims

1. Use of a 5-HT₃ receptor antagonist for the manufacture of a pharmaceutical composition for the treatment of diseases caused or influenced by activation of thrombocytes.

2. The use of a 5-HT₃ receptor antagonist according to claim 1 for the treatment of myocardial infarction, stroke, thrombosis or atherosclerosis.

3. The use of a 5-HT₃ receptor antagonist according to claim 1 for the treatment of myocardial infarction.

4. The use of a 5-HT₃ receptor antagonist according to claim 1 for the treatment of stroke.

5. The use of a 5-HT₃ receptor antagonist according to claim 1 for the treatment of thrombosis.

6. The use of a 5-HT₃ receptor antagonist according to claim 1 for the treatment of atherosclerosis.

7. The use according to claim 1 wherein the 5-HT₃ receptor antagonist is selected from the group consisting of ondansetron, granisetron, dolasetron, tropisetron, ramosetron, fabesetron, lintopride, alosetron, cilansetron, palonosetron, azasetron and zatosetron, in free form or as a pharmaceutically acceptable salt.

8. The use according to claim 1 wherein the 5-HT₃ receptor antagonist is tropisetron.

9. A method for treatment of diseases caused or influenced by activation of thrombocytes in a subject in need of such treatment, which comprises administering to said subject a therapeutically effective amount of a 5-HT₃ receptor antagonist.

10. A pharmaceutical composition which incorporates as active agent a 5-HT₃ receptor antagonist for use in the treatment of diseases caused or influenced by activation of thrombocytes.
